# EUROPEAN PATENT APPLICATION

(11) **EP 1 671 623 A1**
(43) Date of publication of application: **21.06.2006**
(21) Application number: 04023474.2
(22) Date of filing: 01.10.2004
(51) Int. Cl.: A61K 8/49, A61Q 11/00, A61K 31/415, A61K 31/7056

(54) **Composition for use in the antibacterial and antiinflammatory treatment of the oral cavity**

(71) Applicant: Vrespa, Giuseppe, I-20025 Legnano (Milan) (IT)
(72) Inventor: Vrespa, Giuseppe, 20025 Legnano (MI) (IT); Smojver, Erica, 24100 Bergamo (BG) (IT)
(74) Representative: Gervasi, Gemma

(57) **Abstract**

The present invention refers to a new composition for use in the anti-inflammatory and antibacterial treatment of the oral cavity, comprising a synergic mixture of Metronidazole and Clindamycin. The invention further relates to a toothpaste formulation comprising a synergic mixture of those two components.

## Description

### Field of the invention

The present invention refers to a new composition for use in the anti-inflammatory and antibacterial treatment of the oral cavity, comprising a synergic mixture of metronidazole and clindamycin.

### State of the art

The metronidazole, i.e. 1-(2-hydroxyethyl)-2-metyl-5-nitroimidazole, is an antibiotic which recently has found an application in various pharmacological fields. It is particularly active as well against anaerobic bacteria like *Bacterioides, Fusobacteria, Actinobacillus Actinomicetem comitans,* as on anaerobic Gram positive cocci.

In medicine the metronidazole is used for systematic administrations against serious inflammatory diseases like septicaemia, cerebral abscess, necrotic pneumonia, osteomielyte, pelvic abscess, peritonitis, post-surgical wounds and for the prevention of post-surgical inflammations.

The most common use for the compound is linked, in any case, to skin pathologies like e.g. the acnae *rosacea,* against which it is particularly active.

In dentistry the metronidazole is used with systematic administration (25% gel in combination with others antibiotics like amoxicillin) as a coadjutant for the treatment of parodontal diseases in the acute phase.

The bacteria which are responsible of such pathology are mainly Gram-negative anaerobes, like e.g. *Actinobacillus Actinomicetemcomitans, Bacterioides Phorsytus, Campilobacter rectus, Eubacterium nodatum, Fusobacterium nucleatum, Peptostreptococcum micros, Porphiromonas gingivalis and Prevotella intermedia.* Furthermore, also G+ cocci can be observed, like *Streptococcus intermedius.*

Against those bacteria the metronidazole has shown a particular efficacy, and in fact it has high selectivity and specificity.

In case of a systematic and therefore continuous administration of this compound, the most common problem is the appearance of side-effects due to intolerance towards high concentrations of active substance. Those phenomena regard principally the gastrointestinal area and for this reason the administration of metronidazole is limited to the topic use, thus avoiding the oral administration.

The Clindamycin is considered the first-choice antibiotic for the treatment of dental inflammations. It is particularly active against Gram-positive cocci (with the exception of enterococci) and in particular in the case of streptococci and many Gram-negative anaerobic obliged bacteria which colonise the root canal of necrotic teeth.

On the other hand, Clindamycin is not effective against the mycoplasm and the Gram-negative facultative bacteria. The action of Clindamycin derives from the inhibition of the protein synthesis due to the bond formed with the ribosome sub-unity 50S of bacteria. By high concentrations, it has a bactericide effect.

Usually, this compound is administered orally since in this way it has been found that 90% of the compound can be quickly assimilated. The assimilation is not relevantly influenced by the presence of food, and the characteristic blood-peak can be observed after 45-60 minutes from the administration.

Due to its low molecular weight the Clindamycin diffuses rapidly within the tissues, and in particular in bone tissue. The metabolization occurs principally in the liver, and the elimination is by urine and in the bile.

Hypersensitization reactions are rare and they are normally revealed by cutaneous eruptions.

The main problem derived by the systematic use of Clindamycin resides in the fact that its administration causes pseudomembranouse colitis with diarrhoea. The symptoms are due to disequilibrium of the intestinal bacterial flora which leads to the proliferation of the *Clostridium difficilis* producing an enterotoxine responsible of the colitis.

The use of a mixture of Metronidazole and Clindamycin is described in the patent US 5,849,776 where the combination of those compounds results in a synergic mixture used in the treatment of skin diseases and is limited to the topic application. In this document, there are no indications at all about the possibility of using the combined action of the bacterial-specific selectivity of Metronidazole and the efficacy of Clindamycin for the cure of bacterial infections and inflammations of the oral cavity.

### Scope of the invention

On the basis of what has been said above, it is then a principal scope of the present invention to provide an antibacterial and anti-inflammatory composition for the oral cavity which can be used without incurring in the intolerance phenomena cited above due to the systematic administration.
Another object of the present invention is also to provide an antibacterial and anti-inflammatory formulation which can be used in the various pharmacological forms.

### Description of the invention

The solution to the problems constituting the objects of the present invention resides in a formulation comprising a synergic mixture of Metronidazole and Clindamycin to be employed in the antibacterial and anti-inflammatory treatment of the oral cavity according to claim 1.

Other advantages and specific aspects of the invention are defined in claim 8 and in the dependent claims.

The above-mentioned mixture has been revealed as being particularly effective in the treatment of infections from bacterial species responsible of parodontal pathologies, which, as many studies have demonstrated, are the same found in periimplantitis. It has been shown, however, that in case of periimplantitis the inflammation involves also the alveolar bone, while in case of parodontitis the inflammation is limited to the connective tissue.

The composition according to the present invention finds an application specifically for the combined action against both inflammatory diseases and is characterised by efficacy and selectivity. In the advanced phase, in fact, the odontogenous infection is carried mainly by anaerobic obliged bacteria. It has been demonstrated that these bacteria in the late inflammatory phase show a resistance to penicillin in the 35% - 50% of the cases.

For this reason, a few days before the appearance of the first symptoms the Clindamycin represents the first-choice compound. In case of infections for which penicillin is inactive, the association of Metronidazole with Clindamycin results in an effective action against obliged anaerobic bacteria. Odontogenous infections are always the result of a combined effect of obliged anaerobic and facultative aerobic bacteria and Metronidazole alone is therefore not effective, while the combination Metronidazole Clindamycin is the new complete and quick solution for a complete and functional treatment.

The formulation comprising Metronidazole and Clindamycin according to the present invention which has shown the best results and which has the best versatility for all the pharmacological applications directed to the anti-inflammatory and antibacterial treatment of the oral cavity, comprises:
- from 0.01 to 0.1% of clindamycin,
- from 0.01 to 0.1% of metronidazole,
- from 5 to 10% of propylene glycol,
- from 1 to 10% of a 70% solution of sorbitol,
- from 0.1 to 0.5% of sodic salt of dymethylparaoxybenzoate,
- a pharmaceutically suitable carrier.

This composition represents the basis for the preparation of every pharmaceutical product intended for the local treatment, thus avoiding the contraindications linked to enteral and parenteral administrations.

To this scope, the formulation according to the present invention is particularly effective if applied in the form of a mouthwash solution, a spray or tablets and in particular lozenge.

Furthermore, according to another aspect of the invention, the synergic effect of Metronidazole and Clindamycin is optimal if applied in the formulation of a toothpaste. This comprises:
- from 0.01 to 0.1% of clindamycin,
- from 0.01 to 0.1% of metronidazole,
- from 5 to 10% of propylene glycol,
- from 1 to 10% of a 70% solution of sorbitol,
- from 0.1 to 0.5% of sodic salt of dymethylparaoxybenzoate,
- from 1 to 5% of vegetal glycerol
- from 1 to 5% of hydroxyethylcellulose
- a pharmaceutically acceptable carrier

This toothpaste can be used as well for the daily use as for the systematic treatment during the acute inflammatory phase. It can be also combined with the usual treatments for the cure of periimplantitis.

It has been found that it is also advantageous to use it periodically (one week per month) as a means for controlling and re-equilibrating the bacterial flora since it limits the formation of anaerobic bacteria which are potentially responsible of periimplantitis.

In addition to the cited compounds, all the above-listed pharmaceutical products comprise also pharmaceutically suitable additives and/or commonly used cosmetic compounds.

### Description of the figures

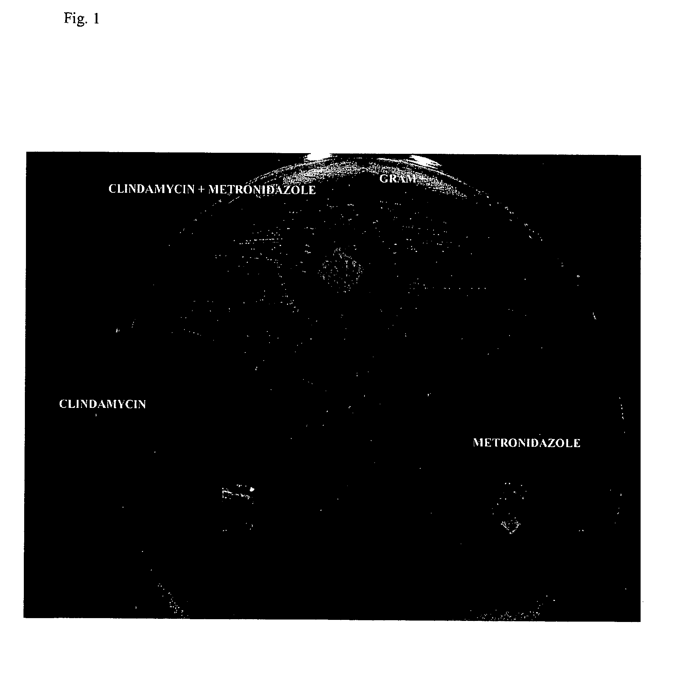

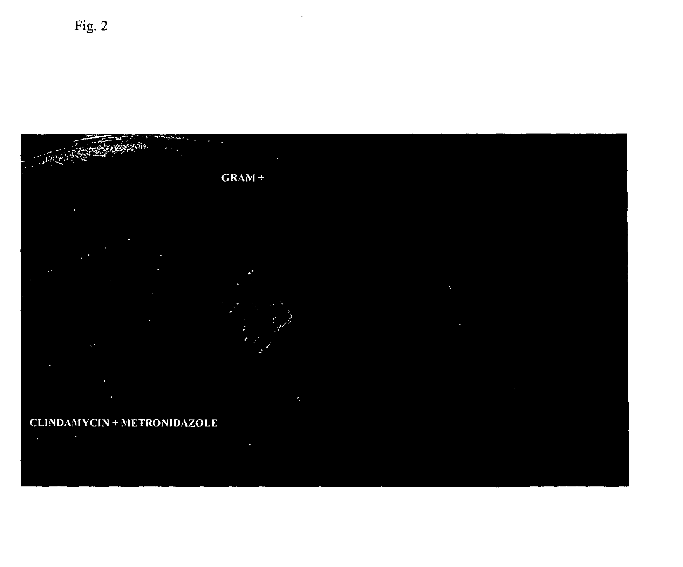

Figures 1 and 2 show the tests regarding the action on the Gram+ bacteria of a composition according to the present invention, comprising a mixture of Metronidazole and Clindamycin.

## Claims

1. Use of a composition comprising a synergic mixture of metronidazole and clindamycin for the preparation of an anti-inflammatory and anti-bacterial product for the oral cavity.

2. Use of a composition according to claim 1, comprising:
- from 0.01 to 0.1% of clindamycin,
- from 0.01 to 0.1% of metronidazole,
- from 5 to 10% of propylene glycol,
- from 1 to 10% of a 70% solution of sorbitol,
- from 0.1 to 0.5% of sodic salt of dymethylparaoxybenzoate,
- a pharmaceutically suitable carrier.

3. Use of a composition according to claim 1 or 2, Wherein said product is a mouthwash solution.

4. Use of a composition according to claim 3, wherein said mouthwash solution is in the form of a spray.

5. Use of a composition according to claim 1 or 2, wherein said product is in the form of tablets.

6. Use of a composition according to one of the claims 1 to 5, as well for the daily use as for the periodic systematic treatment.

7. Toothpaste for antibacterial and anti-inflammatory use comprising a synergic mixture of metronidazole and clindamycin.

8. Toothpaste according to claim 7, comprising:
- from 0.01 to 0.1% of clindamycin,
- from 0.01 to 0.1% of metronidazole,
- from 5 to 10% of propylene glycol,
- from 1 to 10% of a 70% solution of sorbitol,
- from 0.1 to 0.5% of sodic salt of dymethylparaoxybenzoate,
- from 1 to 5% of vegetal glycerol
- from 1 to 5% of hydroxyethylcellulose
- a pharmaceutically acceptable carrier
